Europäisches Patentamt

European Patent Office

Office européen des brevets

(19)

(11) Veröffentlichungsnummer : **0 011 776**
**B1**

(12)

# EUROPÄISCHE PATENTSCHRIFT

(45) Veröffentlichungstag der Patentschrift :
07.04.82

(51) Int. Cl.³ : **A 61 L   2/10, C 02 F   1/32**

(21) Anmeldenummer : **79104507.3**

(22) Anmeldetag : **15.11.79**

(54) Entkeimungsvorrichtung für strömendes Medium.

(30) Priorität : **24.11.78 DE 2851013**

(43) Veröffentlichungstag der Anmeldung :
**11.06.80 (Patentblatt 80/12)**

(45) Bekanntmachung des Hinweises auf die Patenterteilung : **07.04.82 Patentblatt 82/14**

(84) Benannte Vertragsstaaten :
**AT BE CH FR GB IT LU NL SE**

(56) Entgegenhaltungen :
**DE - A - 845 497**
**DE - A - 2 513 429**
**DE - A - 2 622 637**
**FR - A - 1 359 259**
**US - A - 3 923 663**

(73) Patentinhaber : **Katadyn Produkte AG**
**Industriestrasse 27**
**CH-8304 Wallisellen (CH)**

(72) Erfinder : **Keller, Hans Ulrich**
**Albisstrasse 56**
**CH-8038 Zürich (CH)**

(74) Vertreter : **Blum, Rudolf Emil Ernst et al**
**c/o E. BLUM & CO. Vorderberg 11**
**CH-8044 Zürich (CH)**

Imprimerie Jouve, 18, rue St-Denis, 75001 Paris, France

## Entkeimungsvorrichtung für strömendes Medium

Die Erfindung betrifft eine Entkeimungsvorrichtung für strömendes Medium, wie Wasser, mit UV-Röhren, die in einem Ringraum eines Kessels mit Zu- und Ablauföffnungen für das Medium um ein zentrales Rohr herum und parallel dazu angeordnet sind, welches den Kessel durchsetzend an seinem einen Ende eine Auslassöffnung und am anderen Ende eine Einlassöffnung für das Medium aufweist, und von dem Medium im Gegenstrom zur Strömungsrichtung in den Ringraum durchströmt wird.

Derartige Entkeimungsvorrichtungen haben den Zweck, das strömende Medium, wie Wasser, ohne Zusatz von Chemikalien zu entkeimen.

Bei einer bekannten Entkeimungsvorrichtung (Prospekt « Ultra-Violet-Water Purifiers » der Firma Ultra Dynamics Corporation, Paterson, New Jersey, USA), bei welcher der zylindrische Kessel in Längsrichtung von den UV-Röhren, auch « Brenner » genannt, durchsetzt ist, ist die Zulauföffnung gegenüber der Ablauföffnung an axial entgegengesetzten Enden auf in Umfangsrichtung gegenüberliegenden Seiten des Kessel angeordnet. Somit strömt das gesamte zu entkeimende Medium an einer Stelle quer zu den UV-Röhren in den zylindrischen Behandlungsraum ein und auch an einer Stelle quer zu den UV-Röhren wieder aus dem besagten Raum hinaus. Abgesehen davon, dass die Anordnung der UV-Brenner ungünstiger ist, ist die Strömung des Mediums unkontrolliert, wobei die Hauptströmung in der direkten Verbindung zwischen Ein- und Auslass schräg zu den UV-Röhren stattfindet. Ferner ist die Anordnung der UV-Brenner unvorteilhaft. Eine optimale Entkeimung kann somit nicht stattfinden.

Die deutsche Auslegeschrift 1 003 404 zeigt eine Entkeimungsvorrichtung der eingangs beschriebenen Art. Das zu entkeimende Medium wird über ein zentrales Rohr hochgeführt und sprudelt über einen Einlass in einen Ringraum in den Kessel hinunter, in welchem UV-Strahlen auf einem Umfang gleichmässig angeordnet sind. Bei dieser Vorrichtung wird weder eine gleichmässige Bestrahlung des Ringraumes erhalten noch wird der Ringraum gleichmässig von der zu entkeimenden Flüssigkeit durchflossen, so dass es nicht gewährleistet ist, dass jedes Flüssigkeitsquant die gleiche Energiedosis zum Abtöten der Mikroorganismen erhält.

Die deutsche Offenlegungsschrift 1 492 336 zeigt eine Entkeimungsvorrichtung anderer Art mit nur einem zentral angeordneten UV-Strahler.

Die US-Patentschrift 3 865 734 zeigt eine Entkeimungsvorrichtung, die jedoch keine UV-Strahlen benutzt und bei welcher mehrere konzentrische Ringräume mit entsprechenden Ein- und Auslässen vorhanden sind.

Der Erfindung liegt die Aufgabe zugrunde, eine Entkeimungsvorrichtung der eingangs beschriebenen Art so zu verbessern, dass der gesamte ringförmige Bestrahlungsraum mittels der UV-Röhren möglichst gleichmässig bestrahlt und zudem vom zu entkeimenden Medium möglichst gleichmässig durchflossen wird, damit jedes Mediumquant möglichst die gleiche Strahlungsdosis erhält.

Zur Lösung der Aufgabe ist gemäss der Erfindung vorgesehen, dass die UV-Röhren auf Umfängen mit unterschiedlichen Radien im Ringraum verteilt angeordnet und mit ihren einen Enden in Eintrittsöffnungen für das Medium in einer zulaufnahen Kesselwand und mit ihren anderen, nahe der Einlassöffnung gelegenen Enden in einer zulauffernen Kesselwand unterstützt sind, und dass Mittel zum örtlichen Beeinflussen der Mediumströmung am Ende des Ablaufrohres befestigt sind, welche den Ringquerschnitt zwischen Kesselaussenwand und Ablaufrohr überbrücken und mit den Eintrittsöffnungen in der zulaufnahen Kesselwand fluchtende, die UV-Röhren durchlassende Austrittsöffnungen für das Medium aufweisen, und dass ferner die Auslassöffnung des als Ablaufrohr für entkeimtes Medium dienenden Rohres die Ablauföffnung des Kessels bildet.

Indem jedem UV-Brenner eine individuelle Ein- und Ausstrittsöffnung für das Medium in bzw. aus dem Behandlungsraum zugeordnet wird, derart, dass die Strömungsgeschwindigkeit und -richtung optimal beeinflusst werden kann und indem die UV-Brenner auf eine besondere Art angeordnet werden, wird die Entkeimung begünstigt.

Vorzugsweise sind die UV-Röhren über Leitschaufeln abgestützt, welche dem einströmenden Medium eine Zirkulation um die jeweilige UV-Röhre herum erteilen. Damit wird die gegeseitige Lage der Keime bezüglich der UV-Röhren geändert und verhindert, dass Keime im Schatten anderer Keime verbleiben und keine genügende Strahlungsdosis erhalten.

Das zentrale Ablaufrohr führt entkeimtes Medium im Gegenstrom ab und ermöglicht folglich das Vorsehen von Zu- und Ablauföffnungen an einem Ende des Kessels. Dies ist für den Einbau beispielsweise in eine Rohrleitung zweckmässig. In diesem Sinne für die Praxis bevorzugt ist eine Ausführung der Erfindung, bei der die Zu- und Ablauföffnungen in einer gemeinsamen Strömungsachse und die UV-Röhren sowie ein in der zweiten Kammer gelegener gerader Teil des Ablaufrohres unter einem Winkel zu dieser Strömungsachse angeordnet sind und der gerade Teil des Ablaufrohres über einen Rohrkrümmer in der ersten Kammer in die Ablauföffnung übergeht.

Gemäss einer weiteren vorteilhaften Ausgestaltung der Erfindung ist vorgesehen, dass die die UV-Röhren durchlassenden Oeffnungen in Gruppen mit unterschiedlichen Umfängen bezüglich der Mitte des Kessels angeordnet sind und dass die Oeffnungen in der Zwischenscheibe von Gruppe zu Gruppe in Richtung radial nach aussen abnehmenden Durchmesser haben.

Die Erfindung ist im folgenden anhand schematischer Zeichnungen an einem Ausführungsbeispiel mit weiteren Einzelheiten näher erläutert. Es zeigen :

Figure 1 einen Längsschnitt durch eine Entkeimungsvorrichtung gemäss der Erfindung ;

Figure 2 einen im Masstab erheblich verkleinerten und durch Weglassen von Teilen vereinfachten Schnitt nach der Linie II-II in Fig. 1.

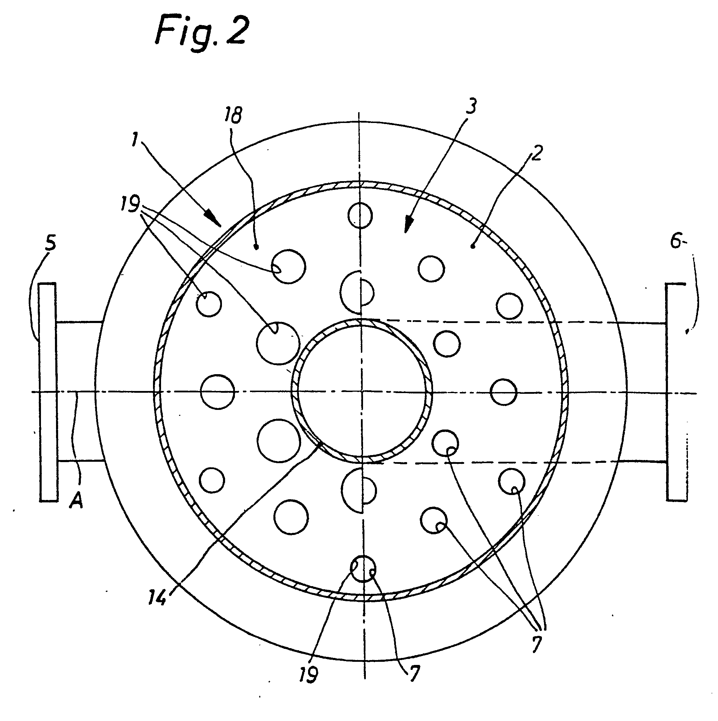

Die gezeigte Entkeimungsvorrichtung umfasst einen zylindrischen Druckkessel 1 mit zwei übereinander angeordneten, durch eine Trennwand 2 getrennten Kammern 3,4. Die untere Kammer 4 weist auf der einen Seite eine Zulauföffnung 5 und auf der anderen Seite eine in der gleichen Strömungsachse gelegene Ablauföffnung 6 auf. Die zylindrisch ausgebildete Kammer 4 ist über die Trennwand 2 durch Eintrittsöffnungen 7 mit der Kammer 3 verbunden. Bei dem gezeigten Ausführungsbeispiel sind insgesamt achtzehn Eintrittsöffnungen vorgesehen. Diese Eintrittsöffnungen sind, wie in der rechten Hälfte der Fig. 2 gezeigt, in drei Gruppen von je sechs Oeffnungen gleichen Durchmessers auf drei unterschiedlichen Umfängen angeordnet. In jeder Eintrittsöffnung ist über je drei Leitschaufeln 8 eine Führung 9 für ein Quarzschutzrohr 10 abgestützt, welches einen schützenden Mantel für eine koaxial darin aufgenommene UV-Röhre bildet (in Fig. 2 nicht gezeichnet). Am oberen Ende ist jedes Quarzschutzrohr über je eine Oeffnung 19 in einer Zwischenscheibe 18 durch Flanschstutzen 11 am Kesseldeckel 12 in eine Schutzhaube 13 durchgeführt, über welche die jeweilige UV-Röhre elektrisch angeschlossen ist. Die UV-Röhren können daher von aussen in den Kessel eingeführt und aus diesem herausgenommen werden.

Die Oeffnungen 19 in der ringförmigen Zwischenscheibe 18 sind mit den Oeffnungen 7 fluchtend angeordnet, haben jedoch von Gruppe zu Gruppe in Richtung radial nach aussen unterschiedlichen, bevorzugt abnehmenden Durchmesser, um die Durchflussmenge pro Zeiteinheit bei jeder Oeffnung 19 so zu beeinflussen, dass die durchfliessenden Wassermengen möglichst die gleiche Strahlungsdosis erhalten. Hierdurch kann erreicht werden, dass an den UV-Röhren vorbeifliessendes strömendes Medium innen, d.h. nahe der Kesselmitte, schneller fliesst als aussen, so dass auch das innen fliessende Medium, wo der Raum besser ausgeleuchtet ist, die gleiche UV-Strahlungsdosis erhält wie aussen fliessendes Medium.

Zentral in der Kammer 3 ist ein vertikaler, gerader Teil 14 eines insgesamt mit dem Bezugszeichen 15 bezeichneten Ablaufrohres angeordnet, der an seinem oberen Ende eine Einlassöffnung 16 aufweist. Das untere Ende des geraden Teiles 14 des Ablaufrohres durchsetzt die Trennwand 2, in welche das Ablaufrohr eingeschweisst ist, und setzt sich unten in die Kammer 4 durch einen Rohrkrümmer 17 fort, der in die Strömungsachse A einmündet und an der Ablauföffnung 6 endet. Die Zwischenscheibe 18 ist mit dem oberen Ende des Ablaufrohres 15 verschweisst.

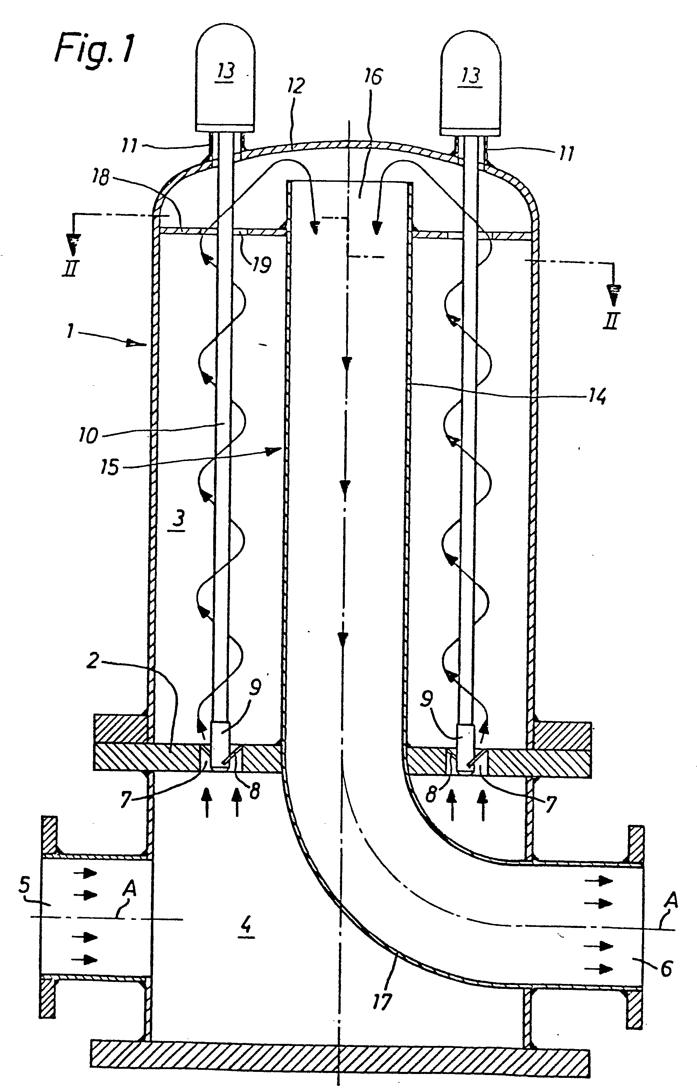

Der Weg des strömenden Mediums ist in Fig. 1 mittels Pfeilen angedeutet. Es ist ersichtlich, dass die Anordnung der UV-Röhren auf unterschiedlichen Umfängen mit einer jeder UV-Röhre koaxial zugeordneten Eintrittsöffnung 7 in Verbindung mit den zwischen der Aussenwand dieser Oeffnung und der Abstützung für die UV-Röhren angeordneten Leitschaufeln 8 zu einer besonders gleichmässigen Entkeimung führt. Das zentrale Ablaufrohr schafft mit dem darin zurückströmenden entkeimten Medium eine Abschirmung vor den im Durchmesser gegenüberliegenden UV-Röhren, so dass radial zwischen dem Teil 14 des Ablaufrohres 15 und den UV-Röhren strömendes Medium nicht stärker bestrahlt wird als radial ausserhalb zwischen den UV-Röhren und der Aussenwand der Kammer 3 strömendes Medium.

Die Entkeimungsvorrichtung ist zur Entkeimung von unter Druck stehendem Medium geeignet. Sie kann beispielsweise für einen Druck von 16 bar ausgelegt sein.

Ein entscheidender Einbauvorteil ist, dass die neue Entkeimungsvorrichtung wegen der gegenüberliegenden Anordnung der Zu- und Ablauföffnungen 5, 6 in einer gemeinsamen Strömungsachse A direkt in die Druckleitung eingebaut werden kann.

Der Ringraum 3 im Bereich zwischen der zulaufnahen Trennwand 2 und der Zwischenscheibe 18 ist frei von strahlungshemmenden Hindernissen, wie Zwischenwände, Umlenkbleche oder dgl., welche üblicherweise vorgesehen werden, um strömende Medien zu beeinflussen. Die erwünschte Strömung bzw. eine Beeinflussung des strömenden Mediums wurde hier durch die den Ringraum begrenzenden Mittel, wie insbesondere die Trennwand 2 und die als Verteilorgan für das zu entkeimende Medium dienende Zwischenscheibe 18, sowie die besondere Anordnung der UV-Röhren bezüglich der Trennwand 2 und der Zwischenscheibe 18, erhalten.

Mit der beschriebenen Vorrichtung wird erreicht, dass unabhängig davon, in welchem Bereich dieses Strömungsraumes das Strömungsmedium strömt, — sei es radial innen oder aussen —, stets eine gleichmässige Entkeimung gewährleistet ist.

## Ansprüche

1. Entkeimungsvorrichtung för strömendes Medium wie Wasser, mit UV-Röhren, die in einem Ringraum eines Kessels mit Zu- und Ablauföffnungen für das Medium um ein zentrales Rohr herum und parallel dazu angeordnet sind, welches den Kessel durchsetzend an seinem einen Ende eine Auslassöffnung und am anderen Ende eine Einlassöffnung für das Medium aufweist, und von dem Medium im Gegenstrom zur Strömungsrichtung in dem Ringraum durchströmt wird, dadurch gekennzeichnet, dass die UV-Röhren (10) auf Umfängen mit unterschiedlichen Radien im Ringraum (3) verteilt angeordnet und mit ihren einen Enden (bei 9) in Eintrittsöffnungen (7) für das Medium in einer zulaufnahen Kesselwand (2) und mit ihren anderen, nahe der Einlassöffnung (16) gelegenen Enden (bei 11) in einer zulauffernen Kesselwand (12) unterstützt sind, und dass Mittel (18, 19) zum örtlichen Beeinflussen der Mediumströmung am Ende des Ablaufrohres (15) befestigt sind, welche den Ringquerschnitt zwischen Kesselaussenwand und Ablaufrohr überbrücken und mit den Eintrittsöffnungen (7) in der zulaufnahen Kesselwand (2) fluchtende, die UV-Röhren durchlassende Austrittsöffnungen (19) für das Medium aufweisen, und dass ferner die Auslassöffnung (6) des als Ablaufrohr für entkeimtes Medium dienenden Rohres (15) die Ablauföffnung des Kessels (1) bildet.

2. Entkeimungsvorrichtung nach Anspruch 1, dadurch gekennzeichnet, dass die UV-Röhren (bei 10) über Leitschaufeln (8), welche dem einströmenden Medium eine Zirkulation um die jeweilige UV-Röhre herum erteilen, in den Eintrittsöffnungen (7) abgestützt sind.

3. Entkeimungsvorrichtung nach Anspruch 1 oder 2, dadurch gekennzeichnet, dass die Zu- und Ablauföffnungen (5, 6) in einer ersten Kammer (4) und die UV-Röhren (bei 10) in einer zweiten Kammer (3) angeordnet sind, und dass die genannte zulaufnahe Kesselwand (2) die Trennwand für die beiden Kammern (3, 4) bildet.

4. Entkeimungsvorrichtung nach Anspruch 3, dadurch gekennzeichnet, dass die Zu- und Ablauföffnungen (5, 6) in einer gemeinsamen Strömungsachse (A) und die UV-Röhren (bei 10) sowie ein in der zweiten Kammer (3) gelegener gerader Teil (14) des Ablaufrohres (15) unter einem Winkel zu dieser Strömungsachse (A) angeordnet sind und dass der geraden Teil (14) des Ablaufrohres über einen Rohrkrümmer (1) in der ersten Kammer (4) in die Ablauföffnung (6) übergeht.

5. Entkeimungsvorrichtung nach Anspruch 4, dadurch gekennzeichnet, dass die UV-Röhren (bei 10) und der gerade Teil (14) des Ablaufrohres (15) vertikal und die Strömungsachse (A) horizontal verlaufen und dass die erste Kammer (4) mit den Zu- und Ablauföffnungen (5, 6) unterhalb der zweiten Kammer (3) mit den UV-Röhren liegt.

6. Entkeimungsvorrichtung nach Anspruch 1, dadurch gekennzeichnet, dass die die UV-Röhren (10) durchlassenden Oeffnungen (7, 19) in Gruppen auf unterschiedlichen Umfängen bezüglich der Mitte des Kessels (1) angeordnet sind und dass die Oeffnungen (19) in der Zwischenscheibe (18) von Gruppe zu Gruppe in Richtung radial nach aussen abnehmenden Durchmesser haben.

7. Entkeimungsvorrichtung nach einem der Ansprüche 1 bis 6, dadurch gekennzeichnet, dass das zentrale Ablaufrohr (15) aus einem UV-Strahlung abschirmenden Material besteht.

8. Entkeimungsvorrichtung nach Anspruch 1, dadurch gekennzeichnet, dass der Ringraum (3) im Bereich zwischen der zulaufnahen Kesselwand (2) und der Zwischenscheibe (18) frei von strahlungshemmenden Hindernissen, wie Zwischenwände, Umlenkbleche oder dgl., ist.

## Claims

1. Purification apparatus for a flowing medium such as water, with UV-tubes which are located in an annular space of a vessel having inlet and outlet openings for the medium around a central tube and parallel there-to which extends through said vessel and is provided at one end with an inlet aperture and an outlet aperture for the medium and is flowed through by the medium in a counter flow relative to the direction of flow in the annular space, characterized in that the UV-tubes (10) are distributed along circles of differing diameters within the annular space (3) and are supported by one of their ends (at 9) in inlet apertures (7) for the medium in a wall (2) of the vessel adjacent to the inlet and by their other ends (at 11) located adjacent to the inlet aperture (16) in a wall (2) of the vessel at a distance from the inlet, and in that means (18, 19) for a local influencing of the flow of the medium are mounted to one end of the discharge tube (15) which means bridge the cross-section of the annulus between the vessel outer wall and discharge tube and are provided with outlet openings (19) for the medium aligned with the inlet openings (7) in the wall (2) of the vessel and penetrated by the UV-tubes, and in that the outlet opening (6) of the tube (15) acting as discharge tube for purified medium forms the discharge opening of the vessel (1).

2. Purification apparatus according to claim 1, characterized in that said UV-tubes (at 10) are supported by the agency of guide vanes (8) generating a circulation of the inflowing medium around the respective UV-tube in the inlet openings.

3. Purification apparatus according to claim 1, characterized in that the inlet and outlet openings (5, 6) are arranged in a first chamber (4) and the UV-tubes (at 10) are arranged in a second chamber (3), and in that said wall (2) of the vessel located adjacent the inlet forms the partition for the two chambers (3, 4).

4. Purification apparatus according to claim 3, characterized in that the inlet and outlet openings (5, 6) comprise a common axis (A) of flow and that the UV-tubes (at 10) as well as a straight section (14) of the discharge tube (15) are arranged at an angle relative to said axis (A) of flow and in that the straight section (14) of the discharge tube leads via an elbow pipe (1) in the first chamber (4) into the discharge opening.

5. Purification apparatus according to claim 4, characterized in that the UV-tubes (at 10) and the straight section (14) of the discharge tube extend vertically and the axis (A) of flow extends horizontally and in that the first chamber (4) with the inlet and outlet openings (5, 6) is located under the second chamber (3) with the UV-tubes.

6. Purification apparatus according to claim 1, characterized in that the openings (7, 19) which are penetrated by the UV-tubes (10) are arranged in groups on the different annular lines raltive to the center of the vessel (1), and that the openings (19) in the intermediate plate (18) have a diameter decreasing from group to group in a radially outwards direction.

7. Purification apparatus according to one of the claims 1 to 6, characterized in that the central discharge tube (15) consists in a material shielding against UV-rays.

8. Purification apparatus according to claim 1, characterized in that the annular space (3) between the wall (2) of the vessel adjacent the inlet and the intermediate plate lacks any ray resisting obstacles such as partitions, guide vanes and similar.

**Revendications**

1. Dispositif de stérilisation pour un milieu qui s'écoule, tel que de l'eau, avec des tubes à UV qui sont disposés dans un espace annulaire d'une enceinte avec des ouvertures d'arrivée et de départ pour le milieu autour d'un tuyau central et parallèlement à celui-ci, lequel tuyau central, traversant l'enceinte, présente à l'une de ses extrémités une ouverture de sortie et à son autre extrémité une ouverture d'entrée pour le milieu, et est parcouru par le milieu, à contre-courant du sens d'écoulement dans l'espace annulaire, caractérisé en ce que les tubes à UV (10) sont répartis dans l'espace annulaire (3) sur des circonférences de rayons différents et sont soutenus par les unes de leurs extrémités (en 9) dans des ouvertures d'entrée (7) pour le milieu, dans une paroi (2) de l'enceinte proche de l'arrivée, et par leurs autres extrémités (11) placées près de l'ouverture d'entrée dans une paroi (12) de l'enceinte, éloignée de l'arrivée, et en ce que des moyens (18, 19) propres à influencer localement l'écoulement du milieu sont fixés à l'extrémité du tuyau de départ (15), ces moyens faisant pont sur la section transversale annulaire entre la paroi extérieure de l'enceinte et le tuyau de départ et présentant des ouvertures de sortie (19) pour le milieu, en regard des ouvertures d'entrée (7) dans la paroi (2) de l'enceinte, proche de l'arrivée, laissant passer les tubes à UV, et en ce qu'en outre l'ouverture de sortie (6) du tuyau (15) servant de tuyau de départ pour le milieu stérilisé forme l'ouverture de départ de l'enceinte (1).

2. Dispositif de stérilisation suivant la revendication 1, caractérisé en ce que les tubes à UV (en 10) sont soutenus dans les ouvertures d'entrée (7) par l'intermédiaire d'aubes directrices (8) qui impartissent au milieu entrant une circulation autour des différents tubes à UV.

3. Dispositif de stérilisation suivant la revendication 1 ou 2, caractérisé en ce que les ouvertures d'arrivée et de départ (5, 6) sont arrangées dans une première chambre (4) et en ce que les tubes à UV (en 10) sont arrangés dans une deuxième chambre (3) et en ce que ladite paroi (2) de l'enceinte, proche de l'arrivée, forme la cloison pour les deux chambres (3, 4).

4. Dispositif de stérilisation suivant la revendication 3, caractérisé en ce que les ouvertures d'arrivée et de départ (5, 6) sont arrangées dans un axe d'écoulement (A) commun et en ce que les tubes à UV (en 10) ainsi qu'une partie rectiligne (14) du tuyau de départ (15), placée dans la deuxième chambre (3), sont arrangés suivant un certain angle avec cet axe d'écoulement (A) et en ce que la partie rectiligne (14) du tuyau de départ passe par un coude de tuyauterie (1) (lire 17) dans la première chambre (4) à l'ouverture de départ (6).

5. Dispositif de stérilisation suivant la revendication 4, caractérisé en ce que les tubes à UV (en 10) et la partie rectiligne (14) du tuyau de départ (15) s'étendent verticalement, et l'axe d'écoulement (A) horizontalement, et en ce que la première chambre (4) avec les ouvertures d'arrivée et de départ (5, 6) se trouve en dessous de la deuxième chambre (3) avec les tubes à UV.

6. Dispositif de stérilisation suivant la revendication 1, caractérisé en ce que les ouvertures (7, 19) laissant passer les tubes à UV (10) sont arrangées en groupes sur des circonférences différentes par rapport au milieu de l'enceinte (1) et en ce que les ouvertures (19) dans le plateau intermédiaire (18) ont des diamètres décroissants, de groupe à groupe, en direction radiale vers l'extérieur.

7. Dispositif de stérilisation suivant l'une des revendications 1 à 6, caractérisé en ce que le tuyau de départ central (15) est fait d'un matériau jouant le rôle d'écran à l'égard du rayonnement UV.

8. Dispositif de stérilisation suivant la revendication 1, caractérisé en ce que l'espace annulaire (3) dans la région comprise entre la paroi (2) de l'enceinte, proche de l'arrivée, et le plateau intermédiaire (18) est dépourvu d'obstacles gênant le rayonnement, tels que des cloisons, des tôles déflectrices ou analogues.

Fig.1

0 011 776

# Fig.2